Europäisches Patentamt

European Patent Office

Office européen des brevets

(11) Veröffentlichungsnummer: **0 373 538 B1**

(12) **EUROPÄISCHE PATENTSCHRIFT**

(45) Veröffentlichungstag der Patentschrift: **10.11.93**

(51) Int. Cl.5: **C07C 29/76**

(21) Anmeldenummer: **89122728.2**

(22) Anmeldetag: **09.12.89**

(54) **Verfahren zur Trennung von Alkohol/Wasser-Gemischen.**

(30) Priorität: **16.12.88 DE 3842299**

(43) Veröffentlichungstag der Anmeldung:
**20.06.90 Patentblatt 90/25**

(45) Bekanntmachung des Hinweises auf die
Patenterteilung:
**10.11.93 Patentblatt 93/45**

(84) Benannte Vertragsstaaten:
**AT BE CH DE ES FR GB GR IT LI LU NL SE**

(56) Entgegenhaltungen:
**WO-A-86/03686
GB-A- 2 201 413
US-A- 4 277 635
US-A- 4 343 623
US-A- 4 487 614**

**ANALYTICAL CHEMISTRY, Band 49, Nr. 12,
Oktober 1977, Seiten 1004A-1014A; E.
GRUSHKA et al.: "Chemically bonded stationary phases in chromatography"**

**JOURNAL OF CHROMATOGRAPHY LIBRARY,
Band 16, 1979, Seiten 206-222, Elsevier
Scientific Publishing Co.; K.K. UNGER:
"Porous silica"**

(73) Patentinhaber: **Kali-Chemie Aktiengesellschaft
Hans-Böckler-Allee 20
D-30173 Hannover(DE)**

(72) Erfinder: **Schwetje, Norbert
Sögelerstrasse 76
D-3000 Hannover 72(DE)**
Erfinder: **Neubüser, Elisabeth
Im Sacke 4
D-3003 Ronnenberg 1(DE)**
Erfinder: **Warmbold, Andreas
Ernst Reuter Strasse 8
D-3163 Sehnde 1(DE)**

(74) Vertreter: **Lauer, Dieter, Dr.
c/o Kali-Chemie Aktiengesellschaft,
Hans-Bockler-Allee 20
D-30173 Hannover (DE)**

JOURNAL OF CHROMATOGRAPHIC SCIENCE, Band 12, Dezember 1974, Seiten 767-778, Preston; R.E. MAJORS et al.: "Studies of siloxane phases bonded to silica gel for use in high performance liquid chromatography"

ULLMANNS ENCYKLOPÄDIE DER TECHNISCHEN CHEMIE, 4. Auflage, Bd. 21, Seite 458-459, Verlag Chemie, Weinheim

**Beschreibung**

Die Erfindung bezieht sich auf ein Verfahren zur Gewinnung von Ethanol aus Ethanol/Wasser-Gemischen umfassend die Verfahrensschritte Strippen, adsorptive Separation und Trocknung an Molekularsieb.

Als Alternative für die destillative Trennung von Lösungsmittelgemischen ist es bekannt, zur Separation der Komponenten des Lösungsmittelgemisches selektive Adsorbentien, z.B. Molekularsiebe, einzusetzen. Adsorbentien können insbesondere dann verwendet werden, wenn die Siedepunkte der zu trennenden Lösungsmittel zu ähnlich sind, bzw. wenn die Komponenten des Lösungsmittelgemisches Azeotrope bilden. Bei einer dieser Techniken läßt man das flüssige Lösungsmittelgemisch durch ein Bett mit Molekularsieb (Zeolith) passieren. Die adsorbierte Komponente, ebenso wie die in den Makroporen und in den interstitiellen Räumen zwischen den Adsorbenspartikeln verbliebene Flüssigkeit werden anschließend mittels Wärmeeinwirkung aus dem Molekularsiebbett (Zeolithbett) desorbiert.

Es wurde ebenfalls vorgeschlagen, ein Dampf- oder Gasphasenverfahren zur Trennung von Lösungsmittelgemischen anzuwenden. Das normalerweise flüssige Lösungsmittelgemisch wird dazu zunächst in die Dampf- bzw. Gasphase überführt und erst in diesem Aggregatzustand dem Adsorptionsbett zur selektiven Adsorption einer oder mehrerer Komponenten zugeführt.

Es ist auch bekannt, vor der adsorptiven Separation der Lösungsmittelgemische zunächst eine der Komponenten des Gemisches durch andere Trennverfahren, z.B. Destillation, anzureichern.

Aus der US-A-4 343 623 ist ein Verfahren zur Abtrennung von Ethanol aus Ethanol/Wasser-Gemischen bekannt, wobei diese Gemische an verestertem Siliciumdioxid adsorbiert und anschließend wieder desorbiert werden. Bei diesem Verfahren wird z. B. ein flüssiges 50:50 Ethanol/Wasser-Gemisch mit dem Adsorbens kontaktiert und anschließend das angereicherte Ethanol mittels Toluol als Desorptionsmittel bei 95 °C unter Druck vom Adsorbens desorbiert. Hiernach muß das Ethanol allerdings vom Desorptionsmittel noch durch nachgeschaltete Destillation getrennt werden.

Nach Gruschka, E. und Kikta, E. J. Jr., Analytical Chemistry, 1977, Nr. 12, Seiten 1004-1014 können sowohl verestertes Siliciumdioxid als auch durch Silanisierung hydrophobiertes Siliciumdioxid als chemisch-gebundene Stationäryhasen in der HPLC-Chromatographie eingesetzt werden. Ein Hinweis auf die Eignung solcher durch Silanisierung hydrophobierter Siliciumdioxide für den Einsatz in der Gewinnung von Ethanol aus Ethanol/Wasser-Gemischen durch separative Adsorption/Desorption findet sich in dieser Literatur allerdings nicht.

Die im Stand der Technik bekannten Verfahren weisen jedoch noch Nachteile auf. Einerseits werden nur unbefriedigende Anreicherungsgrade der gewünschten Komponenten erreicht. Andererseits müssen die bekannten Trennverfahren zur vollständigen Trennung der Komponenten sehr energieaufwendig betrieben werden. Weiterhin erweist es sich als nachteilig, daß bei den bekannten Verfahren die Energieversorgungseinheit, z.B. Kraftwerk, mit den Einheiten der Trenntechnik, z.B. Destillationseinrichtungen, als zentrale gekoppelte Einheiten betrieben werden müssen.

Aufgabe der Erfindung ist es, ein verbessertes Verfahren zur Trennung von Alkohol/Wasser-Gemischen zur Verfügung zu stellen, welches einerseits eine energiesparende Trennung der Komponenten von Alkohol/Wasser-Gemischen unter Verwendung von Adsorbentien mit hohem selektiven Anreicherungsgrad erlaubt, und welches es andererseits gestattet, wenigstens einen Teil der bei Adsorptionsvorgängen freiwerdenden Adsorptionsenergie zu speichern, rückzugewinnen bzw. im Trennprozeß integriert einzusetzen.

Die Aufgabe wird gelöst durch das erfindungsgemäße Verfahren.

Die Erfindung betrifft ein Verfahren zur Gewinnung von Ethanol aus Ethanol/Wasser-Gemischen mit Ethanolgehalten von 5 bis 40 Gew.-%, bei dem man ein flüssiges Ethanol/Wasser-Gemisch durch isothermes Strippen mit Luft bei etwa 20 bis 25 °C in eine gasförmige Mischung aus Ethanol/Wasser/Strippgas überführt und zur adsorptiven Separation bei Normaldruck über ein Adsorbens leitet, welches aus einem amorphen, durch Fällung nach dem Sol-Gel-Prozeß und Tempern erhaltenen, weitporigen Siliciumdioxid-Trägermaterial mit einer Porenweite von 100 bis 200 Angström und mit einer BET-Oberfläche von etwa 300 $m^2/g$ besteht, dessen Oberfläche durch mittels Silanisierung gebundener geradkettiger C8- bis C18-Alkylreste hydrophobiert ist, und von dem man anschließend durch Spülen unter Normaldruck mit bis zu etwa 100 °C heißer Luft ein hochprozentiges Ethanol/Wasser-Gemisch desorbiert und dieses Gemisch anschließend in einer Trocknungsstufe zur vollständigen Entfernung des noch verbliebenen Restwassers mit Molekularsieb 3 Angström behandelt.

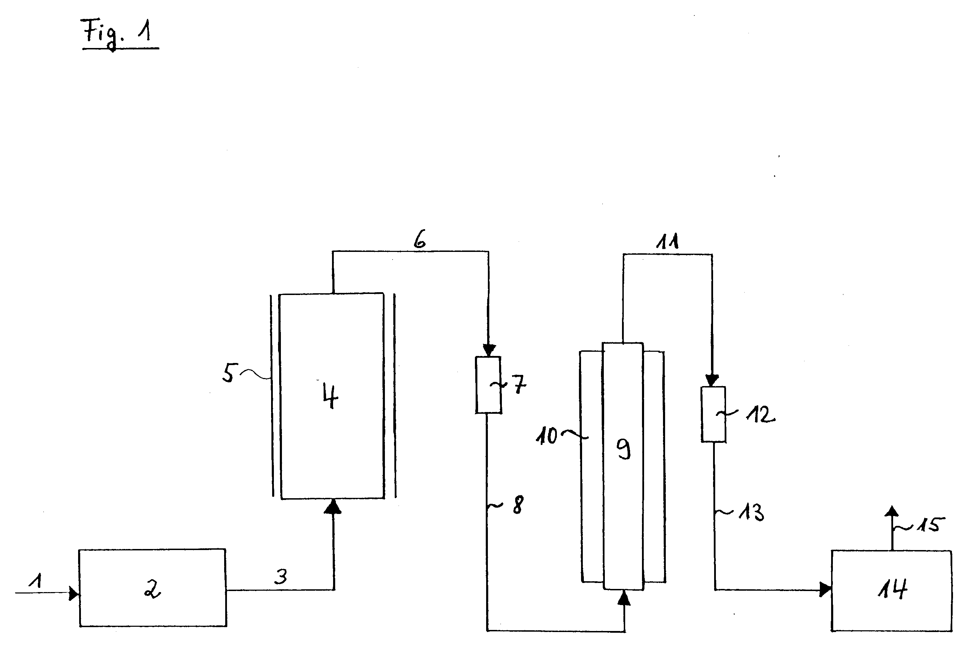

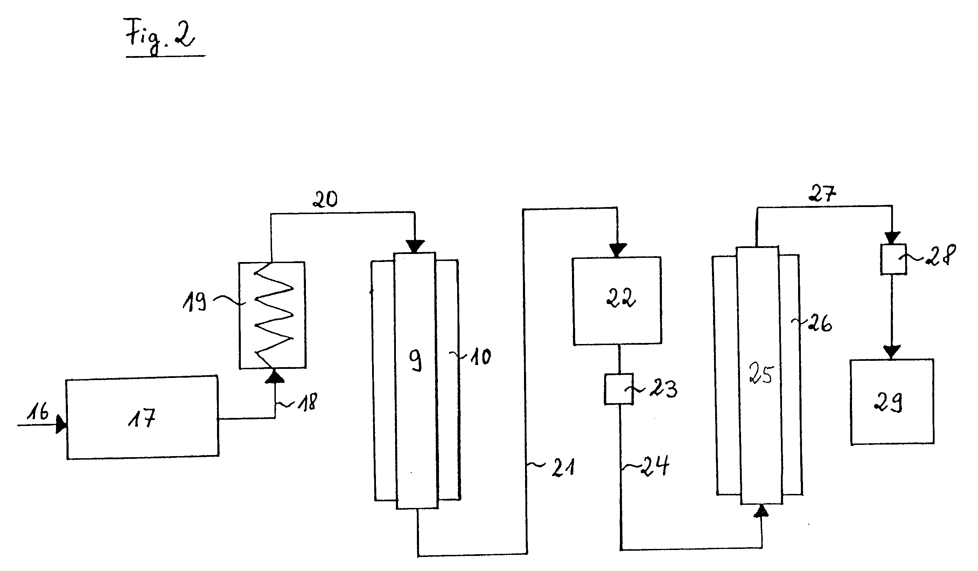

Das erfindungsgemäße Verfahren wird durch die in den Figuren 1 und 2 wiedergegebenen, beispielhaften Fließschemata verdeutlicht.

Figur 1 beschreibt die Verfahrensschritte Strippen und Beladen der adsorptiven Separationseinheit. Zur Durchführung dieser Verfahrensschritte wird mittels einer Pumpe 2 über eine Leitung 1 ein inertes Trägergas (Strippgas) angesaugt und über die Leitung 3 der Strippkolonne 4 zugeführt, welche mit dem zu trennenden Ethanol/Wasser-Gemisch gefüllt ist. Durch Strippen mit dem Trägergas werden zwar die Partialdrücke aller ausdampfenden Komponenten erniedrigt, doch diffundiert hauptsächlich die leichtestflüchtige Komponente (in diesem Fall der Ethanol) in den Trägergasstrom, und zwar auch dann, wenn sie in geringeren Konzentrationen vorliegt. Die Strippkolonne wird isotherm bei ca. 20 bis 25 °C betrieben, d.h. die Verdunstungswärme für das in den Trägergasstrom verdampfende Ethanol/Wasser-Gemisch wird über eine Wärmeaustauschvorrichtung, hier z.B. ein Heizband 5, der Strippkolonne zugeführt. Der die Strippkolonne verlassende, mit gasförmigen Ethanol/Wasser-Gemisch beladene Trägergasstrom gelangt über die Leitungen 6 und 8 in den Adsorber 9. Gegebenenfalls kann der Strippkolonne 5 ein Kühler nachgeschaltet sein, in dem ggf. mitgeführte Flüssigkeitströpfchen aus dem gasförmigen Ethanol/Wasser/Trägergas-Strom abgeschieden werden und in die Strippkolonne 5 zurückfließen; gleichzeitig kann dann der mit gasförmigem Ethanol/Wasser-Gemisch beladene Trägergasstrom im Kühler temperiert werden, z.B. auf 20 °C. Im Adsorber 9 wird aus dem zugeführten Gasgemisch bevorzugt Ethanol an ein durch Silanisierung hydropho-biertes erfindungsgemäßes Adsorbens auf Siliciumdioxid-Basis adsorbiert, dadurch angereichert und von einem Teil des Wassers befreit. Die Adsorption wird isotherm geführt und die freiwerdende Adsorptionswärme daher über eine Wärmeaustauschvorrichtung 10 abgeführt; der Adsorber wird auf etwa 20 °C temperiert. Das den Adsorber durch die Leitung 11 verlassende gasförmige Gemisch aus nicht adsorbiertem Wasser und Trägergas wird über Leitung 13 einer zur Kontrolle des Durchsatzes dienenden Gasuhr 14 zugeführt und abschließend über Leitung 15 entlassen.

Während des Beladungsvorganges (selektive Adsorption) des Adsorbens wird die Zusammensetzung des Gasgemisches vor und nach Adsorber 9 ständig kontrolliert. Hierzu bedient man sich der Probeentnahmevorrichtungen 7 und 12, durch die Gasproben aus dem Trägergasstrom entnommen werden, um darin die Ethanol- bzw. Wasser-Anteile, z.B. gaschromatographisch, zu bestimmen.

Die Schüttung des Adsorbens im Adsorber 9 verhält sich während des Beladungsvorganges so, daß der Adsorptionsvorgang sich mit fortschreitender Beladung vom Eingang in Strömungsrichtung in die frische (oder regenerierte) Adsorptionsmittelschicht verlagert. Zwischen dem beladenen und nicht beladenen Teil der Schüttung bildet sich eine Übergangszone, die sogenannte Adsorptionszone, aus, die im Verlaufe der Beladung des Adsorbens durch die ganze Schüttung wandert. Vor und hinter der Adsorptionszone sind Gaskonzentrationen und Beladung konstant und miteinander im Gleichgewicht. Innerhalb der Adsorptionszone herrscht kein Adsorptionsgleichgewicht. Wenn die Adsorptionszone das Ende der Adsorptionsmittelschicht erreicht, macht sich das in einer Erhöhung der Konzentration an bevorzugt adsorbierter Komponente, hier Ethanol, im durch Leitung 11 austretenden Gasstrom bemerkbar. Mit diesem sogenannten Durchbruch ist die Kapazität des Adsorbers erschöpft und der Beladungsvorgang wird abgebrochen. Bei diskontinuierlichem Betrieb müssen die voranstehenden Verfahrensschritte unterbrochen werden, bis der Adsorber entladen bzw. regeneriert ist und für eine erneute Beladung wieder zur Verfügung steht. Bevorzugt ist jedoch der kontinuierliche Betrieb; hierzu kann das durch Leitung 8 anströmende Gasgemisch z.B. auf einen zweiten Adsorber mit frischem Adsorptionsmittel geschaltet werden, während der erste Adsorber regeneriert, d.h. vom angereicherten Ethanol/Wasser-Gemisch entladen wird.

Figur 2 beschreibt die Verfahrensschritte der Entladung/Regenerierung des erschöpften Adsorbens im Adsorber 9 und die vollständige Trocknung des dabei gewonnenen konzentrierten, noch Restwasser-haltigen Ethanols.

Zur Durchführung der Regenerierung (Desorption) wird der Absorber 9 mit einem beliebigen inerten Gas, vorzugsweise mit Luft, gespült. Hierzu wird das Gas mittels einer Pumpe 17 über eine Leitung 16 angesaugt und über die Leitungen 18 und 20 dem Adsorber 9 zugeführt. Die Desorption wird durch Temperaturerhöhung begünstigt. Es ist daher vorteilhaft ein heißes Spülgas zu verwenden; demgemäß wird das Spülgas zunächst in einer Wärmeaustauschereinheit 19 erwärmt, bevor es über Leitung 20 zum Adsorber 9 gelangt. Bei Adsorbern, die mit Kühlrohren zur Abführung der Adsorptionswärme ausgerüstet sind, kann mit diesen Wärmeaustauschflächen das Adsorbens bei der Regenerierung zusätzlich aufgeheizt werden. Über Leitung 21 verläßt ein Gasgemisch aus Spülgas, Ethanol und Restwasser den Adsorber 9 und gelangt in den Kondensator 22, wo Ethanol und Restwasser aus dem Spülgas auskondensiert werden. Der kondensierte, Restwasser-haltige Ethanol wird über Leitung 24 einem mit Molekularsieb 3 Angström gefüllten Adsorber 25 zugeführt und dort vom Restwasser befreit. Die im Adsorber 25 freiwerdende Adsorptionswärme kann gespeichert oder über eine Wärmeaustauschvorrichtung 26 abgeführt werden. Über Leitung 27 verläßt nunmehr wasserfreier Ethanol den Adsorber 25 und wird in einem Vorratsbehälter 29 gesammelt. Während des Desorptionsvorganges wird die Zusammensetzung des im Kondensator 22 gebildeten Kondensates nach Entnahme über die Probeentnahmevorrichtung 23 kontrolliert. Gleichfalls kontrolliert man die Reinheit des gewonnenen Ethanols nach Entnahme über die Probeentnahmevorrichtung 28.

Sobald der Adsorber 9 regeneriert ist, steht er für eine erneute Beladung zur Verfügung und kann wieder dem oben beschriebenen Beladungsverfahren zugeschaltet werden.

Die durch Entladung/Regeneration des Adsorbers 9 gewonnenen aufkonzentrierten Ethanol/Wasser-Gemische können zur vollständigen Entfernung von Restwasser (Trocknung) direkt mit Molekularsieb 3 Angström behandelt werden. Es ist aber auch möglich, die dem Adsorber 9 entstammenden Ethanol/Wasser-Gemische zuvor durch erneutes Strippen und/oder erneute adsorptive Separation weiter mit Ethanol anzureichern und erst dann der Behandlung mit Molekularsieb 3 Angström zu unterwerfen.

Die Adsorption von Restwasser an Molekularsieb 3 Angström im Adsorber 25 verläuft in analoger Weise zu dem oben für Absorber 9 geschilderten Adsorptionsvorgang. Sobald die Kapazität des Molekularsiebadsorbers erschöpft ist, muß die Trocknung des Ethanols unterbrochen und das Molekularsieb regeneriert werden. Vorteilhafter ist jedoch, z.B. auf einen zweiten Adsorber mit frischem Molekularsieb umzuschalten, während der erste Adsorber regeneriert wird, um so einen kontinuierlichen Betrieb zu gewährleisten. Das im Adsorber 25 enthaltene Molekularsieb 3 Angström wird nach an sich bekannten Methoden regeneriert, d.h. vom adsorbierten Wasser und zusätzlich anhaftenden Ethanolresten befreit; hierzu leitet man z.B. einen möglichst trockenen, heißen Luftstrom über das im Adsorber 25 enthaltene Molekularsieb.

Nach dem erfindungsgemäßen Verfahren lassen sich Ethanol/Wasser-Gemische trennen, bei denen das Verhältnis der Komponenten Ethanol und Wasser in einem weiten Bereich von 5 bis 40 Gew.-% Ethanolgehalt variierbar ist.

In einer bevorzugten Variante des erfindungsgemäßen Verfahrens werden verdünnte Ethanol/Wasser-Gemische mit Alkoholgehalten von 5 bis 15 Gew.- %, vorzugsweise von 7 bis 12 Gew.-%, eingesetzt. Insbesondere können nach dieser Variante des erfindungsgemäßen Verfahrens als verdünnte Ethanol/Wasser-Gemische durch mikrobiologische Prozesse gewonnene Ethanol/Wasser-Lösungen nach Abtrennung von der Biomasse eingesetzt werden.

In vorteilhaften Ausgestaltungen des erfindungsgemäßen Verfahrens werden durch Strippen angereicherte gasförmige Gemische aus Ethanol, Wasser und Strippgas mit Ethanolgehalten von >20 Gew.-% dem Verfahrensschritt der adsorptiven Separation zugeführt. Vorzugsweise weist dieses durch Strippen angereicherte gasförmige Ethanol/Wasser/Strippgas-Gemisch Ethanolgehalte von 40 bis 60 Gew.-% auf.

In einer vorteilhaften Variante des erfindungsgemäßen Verfahrens wird durch die sich an den Strippvorgang anschließende Stufe der adsorptiven Separation das Ethanol/Wasser-Gemisch auf einen Ethanolgehalt von 55 bis 90 Gew.-%, angereichert. Vorzugsweise wird auf Ethanolgehalte von 60 bis 80 Gew.-% angereichert.

Die adsorptive Separation wird mit durch Silanisierung hydrophobierten Adsorbentien auf Siliciumdioxid-Basis durchgeführt, wobei das für die Silanisierung eingesetzte Trägermaterial aus einem amorphen, durch Fällung nach dem Sol-Gel-Prozeß und Tempern erhaltenen, weitporigen Siliciumdioxid mit einer BET-Oberfläche von etwa 300 m$^2$/g und mit einer Porenweite (mittlerer Porendurchmesser) von 100 bis 200 Angström besteht. Besonders vorteilhaft lassen sich als Trägermaterial Siliciumdioxide mit einer Porenweite von 125 ± 15 Angström verwenden. In einer zweckmäßigen Ausgestaltung des erfindungsgemäßen Verfahrens werden solche durch Silanisierung hydrophobierte Adsorbentien eingesetzt, welche durch Umsetzung des Siliciumdioxidträgermaterials mit einer 5 bis 30 Gew.-%igen, vorzugsweise 5 bis 15 Gew.-%igen, Lösung eines Alkyltrimethoxysilans mit einem geradkettigen C8- bis C18-Alkylrest in einem organischen Lösungsmittel und nachfolgende Trocknung unter Erwärmung erhalten wurden. Als organische Lösungsmittel für die als Silanisierungsmittel eingesetzten Alkyltrimethoxysilane können an sich alle organischen Lösungsmittel Verwendung finden, in denen die Alkyltrimethoxysilane gut löslich sind, z.B. aliphatische und aromatische Kohlenwasserstoffe, Chloralkane, Alkohole, Ether oder Ketone. Bevorzugtes Lösungsmittel ist Aceton.

Günstige Alkyltrimethoxysilane mit geradkettigen C8- bis C18-Alkylresten sind Octyltrimethoxysilane, Decyltrimethoxysilan, Dodecyltrimethoxysilan oder Octadecyltrimethoxysilan. Insbesondere erweist sich aber Octyltrimethoxysilan als besonders vorteilhaft.

Die in einzelnen Verfahrensschritten, z.B. beim Strippen oder bei der Regenerierung, benötigte Energie kann in Form thermischer Energie unterschiedlicher Herkunft zugeführt werden. Es kann sowohl in üblicher Art mittels Primärenergie oder Elektrizität direkt beheizt werden oder es kann Abwärme ausgenutzt werden, z.B. von konventionellen Heizsystemen oder Verbrennungsmotoren, oder es kann Energie aus der Umwelt, z.B. Solarenergie, entweder direkt oder nach Transformation mittels einer Wärmepumpe ausgenutzt werden.

In einer Variante des erfindungsgemäßen Verfahrens wird zum Strippen und/oder für die Desorption des hochprozentigen Ethanol/Wasser-Gemisches in der Separationsstufe Niedertemperaturwärme, vorzugsweise Solarenergie und/oder industrielle Abwärme, verwendet.

In einer weiteren Verfahrensvariante erweist es sich als vorteilhaft, neben oder anstelle der obigen Energiequellen die abschließende, zur Entfernung von Restwasser mit Molekularsieb 3 Angström betriebene Trockeneinheit gleichzeitig als Energiespeicher für die durch Adsorption des Wassers freiwerdende Enthalpie zu nutzen. Die in der mit Molekularsieb 3 Angström betriebenen Trockeneinheit gespeicherte Energie kann beliebig anderweitig genutzt werden oder wird in einer Ausgestaltung der Erfindung zum Strippen und/oder für die Desorption eines hochprozentigen Ethanol/Wasser-Gemisches vom beladenen Adsorbens der adsorptiven Separationseinheit verwendet.

In einer Ausführungsform des erfindungsgemäßen Verfahrens ist es möglich, die Adsorptions-/Desorptionszyklen der Separationsstufe und/oder die Beladungs-/Regenerationszyklen der mit Molekularsieb betriebenen Trockenstufe als Moving-bed-Verfahren durchzuführen, d.h. Adsorptions- und Desorptionsvorgänge verlaufen kontinuierlich, wobei man das jeweilige Adsorbens im Gegenstromverfahren zum Ethanol/Wasser/Trägergas-Gemisch führt.

Zur Herstellung des im erfindungsgemäßen Trennverfahren verwendeten, durch Silanisierung hydrophobierten Adsorbens auf Siliciumdioxid-Basis wird als Trägermaterial ein amorphes, durch Fällung nach dem Sol-Gel-Prozeß und Tempern erhaltenes Siliciumdioxid mit einer BET-Oberfläche von etwa 300 $m^2/g$ zunächst bei Temperaturen oberhalb 100 °C (z.B. bei 100 - 200 °C), getrocknet, mit einer Lösung eines Alkyltrimethoxysilans in einem organischen Lösungsmittel einige Stunden (z.B. 2 - 6 h) getränkt, das so behandelte Siliciumdioxid von der nicht aufgenommenen Silanlösung abgetrennt und anschließend erneut unter Erwärmung, vorzugsweise bei Temperaturen von 100 bis 200 °C, getrocknet. Hierbei wird im Herstellverfahren als Trägermaterial ein weitporiges Siliciumdioxid mit einer Porenweite von 100 bis 200 Angström eingesetzt. Besonders vorteilhaft lassen sich als Trägermaterial Siliciumdioxide mit einer Porenweite von 125 ± 15 Angström verwenden. Als organische Lösungsmittel für die als Silanisierungsmittel eingesetzten Alkyltrimethoxysilane können an sich alle organischen Lösungsmittel Verwendung finden, in denen die Alkyltrimethoxysilane gut löslich sind, z.B. aliphatische und aromatische Kohlenwasserstoffe, Chloralkane, Alkohole, Ether oder Ketone. Bevorzugtes Lösungsmittel ist Aceton. In einer zweckmäßigen Herstellung des silanisierten Adsorbens auf Siliciumdioxid-Basis werden 5 bis 30 Gew.-%ige, vorzugsweise 5 bis 15 Gew.-%ige, Lösungen eines Alkyltrimethoxysilans mit einem geradkettigen C8- bis C18-Alkylrest in den angegebenen Lösungsmitteln verwendet. Für die Silanisierung geeignete Alkyltrimethoxysilane sind Octyltrimethoxysilan, Decyltrimethoxysilan, Dodecyltrimethoxysilan oder Octadecyltrimethoxysilan. Als besonders günstig erweist sich Octyltrimethoxysilan.

Es werden durch Silanisierung hydrophobierte Adsorbentien erhalten, die einen durch Elementaranalyse ermittelten Kohlenstoffgehalt von 2 bis 7 Gew.-%, vorzugsweise von 2 bis 4 Gew.-% (bezogen auf das Gesamtgewicht des Adsorbens) besitzen.

Die vorstehend für das erfindungsgemäße Verfahren hergestellten Adsorbentien weisen eine hohe Wasserfestigkeit auf; sie zeigen weiterhin einen geringen Rauhigkeitsfaktor und somit hohe Abriebfestigkeit. Die in Perlform vorliegenden Adsorbentien sind damit hervorragend als Adsorbens in der Ethanol/Wasser-Trennung verwendbar, insbesondere auch in solchen Varianten des erfindungsgemäßen Trennverfahrens, die nach dem Moving-bed-Verfahren ausgeführt werden.

Für die Herstellung der vorstehend silanisierten Adsorbentien auf Siliciumdioxid-Basis, werden als Ausgangsmaterialien amorphe Siliciumdioxide eingesetzt, die Aluminium-frei sind und eine BET-Oberfläche von etwa 300 $m^2/g$ besitzen und die darüber hinaus weitporig sind, d.h. solche Siliciumdioxide, die mittlere Porendurchmesser von 100 bis 200 Angström, vorzugsweise von 125 ± 15 Angström, aufweisen. Sie werden durch Fällung nach dem an sich bekannten Sol-Gel-Prozeß und nachfolgenden Tempern (z. B. Temperaturen größer 500 °C, vorzugsweise bei 550 bis 650 °C) hergestellt und können als solche für die oben beschriebene Silanisierung eingesetzt oder gewünschtenfalls zuvor noch weiteren Bearbeitungsschritten unterworfen werden. So ist es z.B. auch möglich, die oben erhaltenen, in Perlform vorliegenden Trägermaterialien auf Siliciumdioxidbasis vor der Silanisierung durch Vermahlung in kleinere Korngrößen zu überführen und anschließend unter Zusatz von Kieselsol zu Kügelchen von gewünschter Größe zu granulieren. Das Trägermaterial auf Siliciumdioxid-Basis liegt dann in feinzerteilter Form in einer Matrix aus Kieselsol als Binder vor. Die so erhaltenen Granulate werden bei Temperaturen > 600 °C, vorzugsweise bei 600 bis 700 °C, calciniert und können danach zu den erfindungsgemäßen Adsorbentien silanisiert werden.

Mit dem erfindungsgemäßen Verfahren kann in vorteilhafter und energieschonender Weise in Ethanol/Wasser-Gemischen eine Anreicherung von Ethanol bzw. die Gewinnung von Ethanol aus solchen Gemischen erreicht werden. Die hierzu erforderliche Energie kann in bequemer Weise bereits durch umweltfreundliche Niedertemperaturwärme, z.B. in Form von Solarenergie oder industrieller Abwärme, bereitgestellt werden. Darüber hinaus bietet das Verfahren den Vorteil, daß im Verfahren anfallende Adsorptionswärme gespeichert werden kann bzw. ebenfalls als Energiequelle nutzbar ist. Ein weiterer Vorteil besteht darin, daß das Verfahren in bequemer kontinuierlicher Betriebsweise geführt werden kann. Es läßt sich dezentral, d.h. auch ohne Anwesenheit großer Kraftwerke, auch mit kleinen Energieversorgungseinheiten ausführen und ist somit vom Standort unabhängig, da weniger Voraussetzungen hinsichtlich der Energieversorgung erfüllt sein müssen als es bei Verfahren im Stand der Technik der Fall ist.

Die in der Separationsstufe eingesetzten Adsorbentien auf Basis von durch Silanisierung hydrophobierten Siliciumdioxiden zeigen günstige Anreicherungsgrade hinsichtlich des zu gewinnenden Ethanols bei gleichzeitig leichter und energieschonender Regenerierbarkeit bzw. Desorption. Ein weiterer Vorteil der verwendeten Adsorbentien besteht darin, daß sie aus gut verfügbaren Rohstoffen durch ein einfaches, nicht aufwendiges Verfahren leicht zugänglich und damit auch in großen Mengen gut verfügbar sind. Weiterhin ist die Herstellung dieser Adsorbentien sowie ggf. deren Entsorgung umweltschonend durchführbar.

Beipiel 1:

Nach dem an sich bekannten Sol-Gel-Prozeß hergestellte, wasserhaltige Siliciumdioxid-Perlkörper wurden bei 150 °C zu perlförmigen Siliciumdioxidteilchen getrocknet. Zur Einstellung von spezifischer Oberfläche und Porenweite wurde nachfolgend noch bei 600 °C getempert. Hierdurch wurde ein Siliciumdioxid erhalten, welches eine BET-Oberfläche von 300 m$^2$/g aufwies und dessen mittlerer Porendurchmesser 125 Angström betrug. Weiterhin zeichnete sich das hergestellte Siliciumdioxid durch eine scheinbare Dichte von 0,71 g/cm$^3$, eine wahre Dichte von 2,17 g/cm$^3$ sowie ein Porenvolumen von 0,95 cm$^3$/g aus. Das Siliciumdioxid war Aluminium-frei und besaß etwa 3 bis 4 Oberflächenhydroxylgruppen pro 1 nm$^2$.

Beispiel 2:

50 g des in Beispiel 1 erhaltenen Siliciumdioxides (Korngröße 1 bis 5 mm) mit einer Porenweite von 125 Angström wurde zunächst 16 h bei 160 °C getrocknet. Danach wurden die als Trägermaterial dienenden SiO$_2$-Perlen in einem Kunststoffgefäß 4 h bei Raumtemperatur mit einer 10 Gew.-%igen Lösung von Octyltrimethoxysilan in Aceton derart getränkt, daß die Trägerperlen vollständig von der Silanlösung bedeckt waren. Das so behandelte Siliciumdioxid wurde von der nicht aufgenommenen Silanlösung abgetrennt und anschließend auf einem Sieb in einem Trockenschrank 16 h bei 120 °C getrocknet. Für das erhaltene silanisierte Adsorbens wurde durch Elementaranalyse ein Kohlenstoffgehalt (bezogen auf das Gesamtgewicht des Adsorbens) von 2,83 Gew.-% ermittelt.

In analoger Verfahrensweise zum Beispiel 2 wurden die in der nachfolgenden Tabelle 1 wiedergegebenen silanisierten Adsorbentien mit Trägermaterial auf Siliciumdioxid-Basis hergestellt, sowie durch Elementaranalyse die Kohlenstoffgehalte der erhaltenen silanisierten Adsorbentien ermittelt.

Tabelle 1

| Beispiel | Silan | Konzentration des Silans in Aceton (Gew.-%) | Kohlenstoffgehalt des silanisierten Adsorbens (Gew.-%) |
|---|---|---|---|
| 3 | Si 108 | 5 | 2,07 |
| 4 | Si 108 | 20 | 5,04 |
| 5 | Si 108 | 30 | 7,18 |
| 6 | Si 110 | 10 | -- |
| 7 | Si 112 | 10 | 3,33 |
| 8 | Si 118 | 10 | 4,42 |

Beispiel 9:

Das in Beispiel 1 erhaltene Trägermaterial auf Siliciumdioxidbasis wurde durch Vermahlen zerkleinert. Anschließend wurde dieses zerkleinerte Trägermaterial in an sich bekannter Weise mit Kieselsol zu Korngrößen von 2 bis 5 mm granuliert. Die so erhaltenen Granulatkörner wurden bei einer Temperatur von 600 bis 700 °C calciniert.

Das so erhaltene Trägermaterial wurde analog zu Beispiel 2 mit einer Lösung von Octyltrimethyloxysilan in Aceton behandelt und nach Abtrennung der Silanlösung 16 h bei 120 °C getrocknet.

Beispiel 10:

Das in Beispiel 2 erhaltene, silanisierte Adsorbens wurde in einem Verfahren entsprechend der in den Fig. 1 und 2 wiedergegebenen Verfahrensschemata eingesetzt und in Bezug auf die Trennleistung untersucht.

Hierzu wurde Raumluft mit einer Strömungsgeschwindigkeit von 50 l/h mit Hilfe einer Pumpe 2 angesaugt und einer Strippkolonne 4 zugeführt, welche mit einem Gemisch aus 10 Gew.-% Ethanol und 90 Gew.-% Wasser gefüllt war. Die Strippkolonne 4 wurde isotherm bei ca. 20-25 °C betrieben, d.h. die Verdunstungswärme für das in den Trägergasstrom verdampfende Ethanol/Wasser-Gemisch wurde über ein Heizband 5 der Strippkolonne 4 zugeführt. Der die Strippkolonne verlassende, mit Ethanol/Wasser-Gemisch beladene Luftstrom gelangte zunächst in einen direkt nachgeschalteten Kühler (in Fig. 1 nicht dargestellt), in dem mitgeführte Flüssigkeitströpfchen abgeschieden und gleichzeitig der mit Ethanol/Wasser-Gemisch beladene Trägergasstrom auf 20 °C temperiert wurde. Das den Kühler verlassende gasförmige Gemisch aus Ethanol, Wasser und Luft wurde dem Adsorber 9 zugeführt, der mit dem in Beispiel 2 erhaltenen silanisierten Adsorbens gefüllt war. Vor dem Adsorber 9 befand sich als Probeentnahmevorrichtung 7 eine Gaskuvette mit eingebautem Septum. Hier wurden mit einer Gasspritze Proben des gasförmigen Ethanol/Wasser/Luft-Gemisches entnommen und die Zusammensetzung dieses Gemisches vor Adsorber gaschromatographisch ermittelt. Nach Adsorber 9 befand sich als Probeentnahmevorrichtung 12 ebenfalls eine Gaskuvette mit eingebautem Septum. Hier wurden die Proben nach Adsorber 9 entnommen und deren Zusammensetzung ebenfalls gaschromatographisch ermittelt. Während der Durchführung des Verfahrens wurden alle 15 Minuten vor bzw. nach Adsorber 9 Proben gezogen und gaschromatographisch analysiert. Der Adsorptionsvorgang im Adsorber 9 wurde abgebrochen, sobald diese gaschromatographische Analyse der über 12 entnommenen Proben nach Adsorber den beginnenden Ethanoldurchbruch anzeigten. Die vor Adsorber 9 entnommenen Proben wiesen bis zum Durchbruch eine Zusammensetzung von 40 Gew.-% Ethanol und 60 Gew.-% Wasser auf. Durch das silanisierte Adsorbens (gemäß Beispiel 2) im Adsorber 9 wurden insgesamt 94 Gew.-% des angebotenen Ethanols und 37,2 Gew.-% des angebotenen Wassers adsorbiert. Vom Adsorbens im Adsorber 9 wurde gemäß dem in Fig. 2 dargestellten Verfahrensschritt durch Zufuhr heißer Luft (T ca. 100 °C) über Leitung 20 ein Alkohol/Wasser-Gemisch desorbiert und in der Kondensationseinrichtung 22 kondensiert. Das Kondensat bestand zu 62,8 Gew.-% aus Ethanol und zu 37,2 Gew.-% aus Wasser. Die Aufkonzentrierung von Ethanol im silanisierten Adsorbens im Adsorber 9 betrug damit plus 22,8 Gew.-%.

Solche aufkonzentrierten Alkohol/Wasser-Gemische (hier Ethanol/Wasser-Gemisch) können entweder durch erneutes Strippen und/oder erneute adsorptive Separation weiter mit Alkohol angereichert werden oder zur vollständigen Trocknung mit Molekularsieb 3 Angström behandelt werden. Im vorliegenden Beispiel wurde das kondensierte Ethanol/Wassergemisch dem mit Molekularsieb 3 Angström betriebenen Adsorber 25 zugeführt und wasserfreies Ethanol über Leitung 27 abgezogen und im Vorratsbehälter 29 gesammelt.

Analog zur im Beispiel 10 angegebenen Verfahrensweise wurden die in Tabelle 2 angegebenen silanisierten Adsorbentien untersucht und deren Trennleistung bestimmt. Die durch adsorptive Separation aufkonzentrierten Ethanol/Wassergemisch wurden entweder durch erneutes Strippen und/oder adsorptive Separation weiter mit Ethanol angereichert oder gleich zur vollständigen Trocknung dem Adsorber 25 mit Molekularsieb 3 Angström zugeführt.

Tabelle 2

Alle %-Angaben in Tabelle 2 sind Gew.-%-Angaben.

Tabelle 2

| Bei-spiel Nr. | Adsorbens aus Beispiel Nr. | Zusammensetzung vor Adsorber % Ethanol/% Wasser | Aufnahme von Angebot durch das Adsorbens % Ethanol/% Wasser | Zusammensetzung des vom Adsorbens desorbierten Gemisches Ethanol/Wasser- % Ethanol/% Wasser | Trennleistung = Ethanolan- reicherung |
|---|---|---|---|---|---|
| 11 | 3 | 61,6 / 38,4 | 96,1 / 48,7 | 76,0 / 24,0 | + 14,4 % |
| 12 | 5 | 57,8 / 42,2 | 99,3 / 61,6 | 68,8 / 31,2 | + 11,0 % |
| 13 | 6 | 59,0 / 41,0 | 96,6 / 52,5 | 72,6 / 27,4 | + 13,6 % |
| 14 | 7 | 48,0 / 52,0 | 98,7 / 44,7 | 67,1 / 32,9 | + 19,1 % |
| 15 | 8 | 47,0 / 53,0 | 98,6 / 53,7 | 62,0 / 38,0 | + 15,0 % |
| 16 | 9 | 52,0 / 48,0 | 91,0 / 54,0 | 64,6 / 35,4 | + 12,6 % |

**Patentansprüche**

1. Verfahren zur Gewinnung von Ethanol aus Ethanol/Wasser-Gemischen mit Ethanolgehalten von 5 bis 40 Gew.-%, bei dem man flüssiges Ethanol/Wasser-Gemisch durch isothermes Strippen mit Luft bei etwa 20 bis 25 °C in eine gasförmige Mischung aus Ethanol/Wasser/Strippgas überführt und zur adsorptiven Separation bei Normaldruck über ein Adsorbens leitet, welches aus einem amorphen, durch Fällung nach dem Sol-Gel-Prozeß und Tempern erhaltenen, weitporigen Siliciumdioxid-Trägermaterial mit einer Porenweite von 100 bis 200 Angström und mit einer BET-Oberfläche von etwa 300 m$^2$/g besteht, dessen Oberfläche durch mittels Silanisierung gebundener geradkettiger C8- bis C18-Alkylreste hydrophobiert ist, und von dem man anschließend durch Spülen unter Normaldruck mit bis zu etwa 100 °C heißer Luft ein hochprozentiges Ethanol/Wasser-Gemisch desorbiert und dieses Gemisch anschließend in einer Trocknungsstufe zur vollständigen Entfernung des noch verbliebenen Restwassers mit Molekularsieb 3 Angström behandelt.

2. Verfahren nach Anspruch 1, dadurch gekennzeichnet, daß verdünnte Ethanol/Wasser-Gemische mit Ethanolgehalten von 5 bis 15 Gew.-%, vorzugsweise von 7 bis 12 Gew.-%, eingesetzt werden.

3. Verfahren nach Anspruch 2, dadurch gekennzeichnet, daß ein durch Strippen angereichertes gasförmiges Gemisch aus Ethanol, Wasser und Strippgas mit Ethanolgehalten von > 20 Gew.-%, vorzugsweise von 40 bis 60 Gew.-%, der Separationsstufe zugeführt wird.

4. Verfahren nach Anspruch 1, dadurch gekennzeichnet, daß durch die adsorptive Separation das Ethanol/Wasser-Gemisch auf einen Ethanolgehalt von 55 bis 90 Gew.-%, vorzugsweise 60 bis 80 Gew.-%, angereichert wird.

5. Verfahren nach Anspruch 1, dadurch gekennzeichnet, daß das im Adsorbens als Trägermaterial verwendete Siliciumdioxid ein weitporiges Siliciumdioxid mit einer Porenweite von 125 ± 15 Angström, ist.

6. Verfahren nach Anspruch 1, dadurch gekennzeichnet, daß die Oberfläche des Siliciumdioxid-Trägermaterials durch Silanisierung mit Octyltrimethoxysilan, Decyltrimethoxysilan, Dodecyltrimethoxysilan oder Octadecyltrimethoxysilan hydrophobiert ist.

7. Verfahren nach Anspruch 1, dadurch gekennzeichnet, daß man zum Strippen und/oder für die Desorption des hochprozentigen Ethanol/Wasser-Gemisches in der Separationsstufe Niedertemperaturwärme, vorzugsweise Solarenergie und/oder industrielle Abwärme, verwendet.

8. Verfahren nach Anspruch 1, dadurch gekennzeichnet, daß die abschließende, zur Entfernung von Restwasser mit Molekularsieb 3 A betriebene Trocknungsstufe gleichzeitig als Energiespeicher für die durch die Adsorption des Wassers freiwerdende Enthalpie genutzt wird und die gespeicherte Energie zum Strippen und/oder für die Desorption eines hochprozentigen Ethanol/Wasser-Gemisches vom beladenen Adsorbens in der Separationsstufe verwendet wird.

**Claims**

1. A method for obtaining ethanol from ethanol/water mixtures having ethanol contents of 5 to 40% by weight, in which liquid ethanol/water mixture is converted into a gaseous mixture of ethanol/water/stripping gas by isothermal stripping with air at approximately 20 to 25°C and for adsorptive separation at normal pressure is passed over an adsorbent material which consists of an amorphous, wide-pored silicon dioxide carrier material having a pore width of 100 to 200 angstroms and having a BET surface area of about 300 m$^2$/g and obtained by precipitation according to the sol-gel process and tempering, the surface of which material is rendered water-repellent by straight-chained C8 to C18-alkyl radicals which are bonded by means of silanising, and from which material a high-percentage ethanol/water mixture is then desorbed by scouring under normal pressure with hot air of up to about 100°C and this mixture is then treated in a drying stage for complete removal of the residual water still remaining with a molecular sieve of 3 angstroms.

2. A method according to Claim 1, characterised in that dilute ethanol/water mixtures having ethanol contents of 5 to 15% by weight, preferably of 7 to 12% by weight, are used.

3. A method according to Claim 2, characterised in that a gaseous mixture of ethanol, water and stripping gas which has ethanol contents of > 20% by weight, preferably of 40 to 60% by weight, and has been enriched by stripping is sent to the separation stage.

4. A method according to Claim 1, characterised in that the ethanol/water mixture is enriched to an ethanol content of 55 to 90% by weight, preferably 60 to 80% by weight, by the adsorptive separation.

5. A method according to Claim 1, characterised in that the silicon dioxide used as a carrier material in the adsorbent material is a wide-pored silicon dioxide having a pore width of 125 ± 15 angstroms.

6. A method according to Claim 1, characterised in that the surface of the silicon dioxide carrier material is rendered water-repellent by silanising with octyltrimethoxysilane, decyltrimethoxysilane, dodecyltrimethoxysilane or octadecyltrimethoxysilane.

7. A method according to Claim 1, characterised in that low-temperature heat, preferably solar energy and/or industrial waste heat, is used for stripping and/or for desorption of the high-percentage ethanol/water mixture in the separation stage.

8. A method according to Claim 1, characterised in that the final drying stage which is operated to remove residual water with molecular sieve 3 A is simultaneously used as an energy store for the enthalpy released by the adsorption of the water, and the stored energy is used for stripping and/or for the desorption of a high-percentage ethanol/water mixture of the laden adsorbent material in the separation stage.

**Revendications**

1. Procédé d'obtention d'éthanol à partir de mélanges éthanol/eau ayant des teneurs en éthanol de 5 à 40% en poids, dans lequel on transforme un mélange éthanol-eau liquide, par extraction isotherme avec de l'air à environ 20 à 25°C, en un mélange gazeux éthanol/eau/gaz d'entraînement et le fait passer, en vue d'une séparation adsorptive à la pression normale, sur un adsorbant qui est constitué par une matière-support à base de dioxyde de silicium amorphe, à larges pores, obtenue par précipitation par le procédé solgel et par recuit, ayant un diamètre de pore de 100 à 200 Angströms et une aire BET d'environ 300 $m^2$/g, dont la surface est rendue hydrophobe par des restes alkyles à chaîne droite en $C_8$ à $C_{18}$ liés par silanation et à partir de laquelle on désorbe ensuite un mélange à fort pourcentage d'éthanol/eau par balayage à la pression normale avec de l'air chauffé jusqu'à environ 100°C et on traite subséquemment ce mélange dans une étape de séchage à l'aide d'un tamis moléculaire de 3 Angströms en vue de l'élimination totale de l'eau résiduelle encore présente.

2. Procédé selon la revendication 1, caractérisé en ce que l'on met en oeuvre des mélanges éthanol-eau dilués ayant des teneurs en éthanol de 5 à 15% en poids, de préférence de 7 à 12% en poids.

3. Procédé selon la revendication 2, caractérisé en ce qu'un mélange gazeux d'éthanol, d'eau et de gaz d'entraînement ayant des teneurs en éthanol supérieures à 20% en poids, de préférence de 40 à 60% en poids, enrichi par extraction, est envoyé dans l'étape de séparation.

4. Procédé selon la revendication 1, caractérisé en ce que le mélange éthanol/eau est concentré, par séparation adsorptive, jusqu'à une teneur en éthanol de 55 à 90% en poids, de préférence de 60 à 80% en poids.

5. Procédé selon la revendication 1, caractérisé en ce que le dioxyde de silicium utilisé comme matière-support dans l'adsorbant est un dioxyde de silicium à larges pores ayant un diamètre de pore de 125 ± 15 Angströms.

6. Procédé selon la revendication 1, caractérisé en ce que la surface de la matière-support à base de dioxyde de silicium est rendue hydrophobe par silanation avec de l'octyltriméthoxysilane, du décyltriméthoxysilane, du dodécyltriméthoxysilane ou de l'octadécyltriméthoxysilane.

7. Procédé selon la revendication 1, caractérisé en ce que, pour l'extraction et/ou pour la désorption du mélange à fort pourcentage d'éthanol/eau dans l'étape de séparation, on utilise de la chaleur basse température, de préférence de l'énergie solaire et/ou de la chaleur perdue industrielle.

8. Procédé selon la revendication 1, caractérisé en ce que l'étape de séchage finale conduite avec un tamis moléculaire 3 Å pour l'élimination de l'eau résiduelle sert en même temps de réservoir d'énergie pour l'enthalpie libérée par l'adsorption de l'eau et que l'énergie emmagasinée est utilisée dans l'étape de séparation pour l'extraction et/ou la désorption d'un mélange à fort pourcentage d'éthanol/eau à partir de l'adsorbant chargé.

Fig. 1

Fig.2

EP 0 373 538 B1